# EUROPEAN PATENT SPECIFICATION

(11) **EP 2 989 889 B1**
(45) Date of publication and mention of the grant of the patent: **24.05.2023**
(21) Application number: 14182719.6
(22) Date of filing: 28.08.2014
(51) Int. Cl.: A01H 1/08, A01H 5/00, A01H 5/06, A01H 5/10, C07K 14/415, C12N 15/82

(54) **Generation of haploid plants**
Erzeugung von haploiden Pflanzen
Génération de plantes haploïdes

(43) Date of publication of application: 02.03.2016
(73) Proprietor: KWS SAAT SE & Co. KGaA, 37574 Einbeck (DE)
(72) Inventor: Houben, Andreas, 06484 Quedlingburg (DE); Karimi-Ashiyani, Raheleh, 1648878367 Tehran (IR); Ishii, Takayoshi, 06466 Gatersleben (DE); Stein, Nils, 06484 Quedlinburg (DE); Kumlehn, Jochen, 14469 Potsdam (DE)
(74) Representative: Eisenführ Speiser

(56) References cited:
- WO-A1-2011/044132
- WO-A2-2014/110274
- Inna Lermontova ET AL: "CENH3 for Establishing and Maintaining Centromeres" In: "Plant Centromere Biology", 8 April 2013 (2013-04-08), John Wiley & Sons, Oxford [u.a.], XP055163252, ISBN: 978-1-119-94921-3 pages 67-82, DOI: 10.1002/9781118525715.ch6, * pages 73 and 74 * * *
- MARUTHACHALAM RAVI & SIMON W L CHAN: "Haploid plants produced by centromere-mediated genome elimination", NATURE, NATURE PUBLISHING GROUP, UNITED KINGDOM, vol. 464, no. 7288, 25 March 2010 (2010-03-25), pages 615-620, XP002677783, ISSN: 0028-0836, DOI: 10.1038/NATURE08842
- M. RAVI ET AL: "The Rapidly Evolving Centromere-Specific Histone Has Stringent Functional Requirements in Arabidopsis thaliana", GENETICS, vol. 186, no. 2, 13 July 2010 (2010-07-13) , pages 461-471, XP055142479, ISSN: 0016-6731, DOI: 10.1534/genetics.110.120337
- RAHELEH KARIMI-ASHTIYANI ET AL: "Point mutation impairs centromeric CENH3 loading and induces haploid plants", PROCEEDINGS OF THE NATIONAL ACADEMY OF SCIENCES, vol. 112, no. 36, 20 August 2015 (2015-08-20), pages 11211-11216, XP055233787, ISSN: 0027-8424, DOI: 10.1073/pnas.1504333112
- KALINOWSKA KAMILA ET AL: "State-of-the-art and novel developments of in vivo haploid technologies", THEORETICAL AND APPLIED GENETICS, SPRINGER BERLIN HEIDELBERG, BERLIN/HEIDELBERG, vol. 132, no. 3, 19 December 2018 (2018-12-19), pages 593-605, XP036743630, ISSN: 0040-5752, DOI: 10.1007/S00122-018-3261-9 [retrieved on 2018-12-19]
- BRITT ANNE B ET AL: "Cenh3: An Emerging Player in Haploid Induction Technology", FRONTIERS IN PLANT SCIENCE, FRONTIERS RESEARCH FOUNDATION, CH, vol. 7, 12 April 2016 (2016-04-12), pages 1-10, XP002765862, ISSN: 1664-462X, DOI: 10.3389/FPLS.2016.00357
- JIAOJIAO REN ET AL: "Novel technologies in doubled haploid line development", PLANT BIOTECHNOLOGY JOURNAL, vol. 15, no. 11, 1 November 2017 (2017-11-01), pages 1361-1370, XP055717679, GB ISSN: 1467-7644, DOI: 10.1111/pbi.12805
- JACQUIER NATHANAËL M A ET AL: "Puzzling out plant reproduction by haploid induction for innovations in plant breeding", NATURE PLANTS, NATURE PUBLISHING GROUP UK, LONDON, vol. 6, no. 6, 1 June 2020 (2020-06-01), pages 610-619, XP037171425, DOI: 10.1038/S41477-020-0664-9 [retrieved on 2020-06-08]

## Description

### Generation of haploid plants

The present invention relates to non-transgenic and transgenic plants, preferably crop plants, comprising a nucleotide sequence encoding a centromer histone H3 (CENH3) protein comprising a CATD domain, wherein the nucleotide sequence comprises a mutation causing an amino acid substitution at (a) position 92 of SEQ ID NO: 34; (b) position 106 of SEQ ID NO: 44; or (c) position 4 of SEQ ID NO: 1, wherein the amino acid substitution confers the biological activity of a haploid inducer. Further, the present invention provides methods of generating the plants of the present invention.

The generation and use of haploids is one of the most powerful biotechnological means to improve cultivated plants. The advantage of haploids for breeders is that homozygosity can be achieved already in the first generation after dihaploidization, creating doubled haploid plants, without the need of several backcrossing generations required to obtain a high degree of homozygosity. Further, the value of haploids in plant research and breeding lies in the fact that the founder cells of doubled haploids are products of meiosis, so that resultant populations constitute pools of diverse recombinant and at the same time genetically fixed individuals. The generation of doubled haploids thus provides not only perfectly useful genetic variability to select from with regard to crop improvement, but is also a valuable means to produce mapping populations, recombinant inbreds as well as instantly homozygous mutants and transgenic lines.

Lermontova and Schubert (*First Edition. Edited by Jiming Jiang and James A. Birchler. 2013 John Wiley* & *Sons, Inc. Published 2013 by John Wiley* & *Sons, Inc.*) discuss occurrence regulation, functional conservation, interactions, and potential application of the centromeric histone H3 variant and its peculiarities in plants.

Haploids can be obtained by *in vitro* or *in vivo* approaches. However, many species and genotypes are recalcitrant to these processes. Alternatively, substantial changes of the centromere-specific histone H3 variant (CENH3, also called CENP-A), by swapping its N-terminal regions and fusing it to GFP ("GFP-tailswap" CENH3), creates haploid inducer lines in the model plant *Arabidopsis thaliana* (Ravi and Chan, Nature, 464 (2010), 615-618). CENH3 proteins are variants of H3 histone proteins that are members of the kinetochore complex of active centromeres. With these "GFP-tailswap" haploid inducer lines, haploidization occurred in the progeny when a haploid inducer plant was crossed with a wildtype plant. Interestingly, the haploid inducer line was stable upon selfing, suggesting that a competition between modified and wild type centromere in the developing hybrid embryo results in centromere inactivation of the inducer parent and consequently in uniparental chromosome elimination. As a result, the chromosomes containing the altered CENH3 protein are lost during early embryo development producing haploid progeny containing only the chromosomes of the wildtype parent.

Thus, haploid plants can be obtained by crossing "GFP-tailswap" transgenic plants as haploid inducer to wildtype plants. However, as described above, this technique requires substantial changes of the CENH3 protein and the plants comprise a heterologous transgene, which is economically problematic because of increasing public reluctance toward genetically engineered crops.

It is therefore an object of the present invention to overcome the aforementioned problems and in particular to provide alternative haploid inducer plants which do not comprise substantial modifications of their CENH3 protein and/or which are not genetically engineered.

This problem is solved by the subject matter of the independent claims, in particular a plant having biological activity of a haploid inducer and comprising a nucleotide sequence encoding a centromer histone H3 (CENH3) protein comprising a CATD domain, wherein the nucleotide sequence comprises a mutation causing an amino acid substitution at (a) position 92 of SEQ ID NO: 34; (b) position 106 of SEQ ID NO: 44; or (c) position 4 of SEQ ID NO: 1, wherein the amino acid substitution confers the biological activity of a haploid inducer. The CATD domain of the CENH3 protein corresponds to amino acid sequence from positions 113 to position 155 as set forth in SEQ ID No. 38 derived from *Arabidopsis thaliana* and/or the CATD domain of the CENH3 protein is encoded by a nucleotide sequence corresponding to nucleotides from position 337 to position 465 as set forth in SEQ ID No. 37 derived from *Arabidopsis thaliana.* The *A. thaliana* sequences serve only as references and do not limit the invention to the particular *A. thaliana* sequences. Due to the high level of conservation one skilled in the art is able to find the nucleotide sequence and amino acid sequence corresponding to the *A. thaliana* sequences in any other plant material or plant species.

The mutation causing an amino acid substitution is located within the α2-helix of the CATD domain. The α2-helix corresponds to amino acid sequence from positions 127 to position 155 as set forth in SEQ ID No. 38 derived from *Arabidopsis thaliana* and/or the α2-helix is encoded by a nucleotide sequence corresponding to nucleotides from position 379 to position 465 as set forth in SEQ ID No. 37 derived from *Arabidopsis thaliana.* The *A. thaliana* sequences serve only as references and do not limit the invention to the particular *A. thaliana* sequences. Due to the high level of conservation ones skilled in the art is able to find the nucleotide sequence and amino acid sequence corresponding to the *A. thaliana* sequences in any other plant material or plant species.

CENH3 proteins are variants of H3 histone proteins that are members of the kinetochore complex of active centromeres, i.e. the protein structure on chromosomes where spindle fibres attach during cell division. Basically, CENH3 proteins are characterized by a variable tail domain, which does not form a rigid secondary structure, and a conserved histone fold domain consisting of three α-helical regions, termed α1 to 3, which are connected by loop sections. Within the histone fold domain the highly conserved CATD domain (CENP-A targeting domain) is located, which is formed by parts of the α1-helix, the complete α2-helix and the connecting loop 1. The conserved CATD domain is required for CENH3 loading by chaperones and thus vital for its kinetochore localization and centromere function.

The present inventors surprisingly found that a plant possessing the capability to produce haploid progeny, i.e. a haploid inducer, can be obtained by substituting a single amino acid within the conserved CATD domain in the α2-helix, of the CENH3 protein. Advantageously, this can be achieved by transgenic as well as non-transgenic methods. Non-transgenic methods are preferred because of enormous costs for deregulation of genetically modified organisms (GMO) as well as increasing public rejection of genetically modified organisms (GMO) or plants generated by means of GMO, in particular crops for human consumption, and extensive market authorisation processes including rigorous safety assessments of such GMOs.

The present invention provides a plant having biological activity of a haploid inducer and comprising a nucleotide sequence encoding a centromer histone H3 (CENH3) protein comprising a CATD domain, wherein the nucleotide sequence comprises a mutation causing an amino acid substitution at (a) position 92 of SEQ ID NO: 34; (b) position 106 of SEQ ID NO: 44; or (c) position 4 of SEQ ID NO: 1, wherein the amino acid substitution confers the biological activity of a haploid inducer.

In one embodiment the present invention relates to a plant comprising a nucleotide sequence encoding a centromer histone H3 (CENH3) protein comprising a CATD domain, wherein the part of the nucleotide sequence encoding the CATD domain comprises at least one mutation and wherein the at least one mutation causes an amino acid substitution in the α2-helix which a) is encoded by a nucleotide sequence which corresponds to nucleotides from position 379 to position 465 as set forth in SEQ ID No. 37 derived from *Arabidopsis* thaliana, which corresponds to amino acid sequence from positions 127 to position 155 as set forth in SEQ ID No. 38 derived from *Arabidopsis thaliana,* or having the consensus sequence of SEQ ID No. 1, and b) being positioned within the CATD domain of the CENH3 protein as defined above. Thus, the at least one amino acid substitution is located in the α2-helix of the CATD domain. The non-mutated α2-helix of the CATD domain is highly conserved among plant species and is 29 amino acids long starting with position 1 and ending with position 29. In the present invention, any amino acid position given with respect to the α2-helix or the below described consensus sequence is referring to this numbering system. Preferably, the non-mutated α2-helix exhibits the amino acid sequence as given in Table 1.

**Table 1: Specified amino acids in the α2-helix of the CATD domain**

| Position within the α2-helix | Amino acid(s) |
|---|---|
| 1 | X |
| 2 | X |
| 3 | A |
| 4 | L or V |
| 5 | X |
| 6 | A, C or S |
| 7 | I, L, M or V |
| 8 | H or Q |
| 9 | E |
| 10 | A or S |
| 11 | A, S or T |
| 12 | E |
| 13 | X |
| 14 | X |
| 15 | I, L or V |
| 16 | I or V |
| 17 | X |
| 18 | I, L, M or V |
| 19 | F or L |
| 20 | X |
| 21 | X |
| 22 | X |
| 23 | X |
| 24 | X |
| 25 | C |
| 26 | A, S or T |
| 27 | I, L or V |
| 28 | H |
| 29 | A or S |

More preferably, the α2-helix has the consensus sequence of SEQ ID No. 1, which is

XXALXXXQEXXEXXXXXXFXXXXXCAIHX.

1 10 20 29

As indicated above, the α2-helix comprises unspecified [marked as X] and specified amino acids [marked as one letter code].

An unspecified amino acid as given in Table 1 or in SEQ ID No. 1 is an amino acid which although being specified in a group of particular plant species, in a particular plant genus or in a particular plant species is not conserved in a greater range of plant species. Thus, an unspecified amino acid of SEQ ID No. 1 or as given in Table 1 is in a group of particular plant species, in a particular plant genus or in a particular plant species a well-defined, specific amino acid, which, however, is possibly not found at the same place in another plant species. Thus, an amino acid substitution of an unspecified amino acid of SEQ ID No. 1 or as indicated in Table 1 means that in a plant, namely in a specific plant species, the specific but not conserved amino acid is substituted by another amino acid than naturally occurring at that place in this group of particular plant species, in this particular plant genus or in this particular plant species in the endogenously coded native CENH3 protein of said plant species.

Specified amino acids given in Table 1 and in particular specified amino acids of SEQ ID No. 1 are those which occur in a broad range of plant species, and which are thus well conserved.

In a preferred embodiment, the SEQ ID No. 1 consensus sequence has been compiled from the α2-helix sequences derived from species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas,Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Astragalus sinicus, Lotus japonicus, Torenia fournieri, Allium cepa, Allium fistulosum, Allium sativum,* and *Allium tuberosum.*

In one embodiment, the mutation causes a substitution of a specified amino acid at position 4 of SEQ ID No. 1.

In the context of the present invention, a mutation preferably is a non-synonymous point mutation or substitution in the DNA sequence encoding the CENH3 protein resulting in a change in amino acid. This is also called a missense mutation. Further, the change in amino acid or the amino acid substitution may be conservative, i.e. a change to an amino acid with similar physiochemical properties, semi-conservative, e.g. negative to positively charged amino acid, or radical, i.e. a change to a vastly different amino acid.

In a preferred embodiment of the present invention, the present plant having biological activity of a haploid inducer is homozygous with respect to the at least one mutation. In a further embodiment of the present invention, the present plant having biological activity of a haploid inducer is heterozygous with respect to the at least one mutation.

The plant according to the present invention has the biological activity of a haploid inducer. This means that crossing between the plant according to the present invention and a wildtype plant or a plant expressing wildtype CENH3 protein yields at least 0.1 %, 0.2 %, 0.3 %, 0.4 %, 0.5 %, 0.6 %, 0.7 %, 0.8 %, 0.9 %, preferably at least 1 %, preferably at least 2 %, preferably at least 3 %, preferably at least 4 %, preferably at least 5 %, preferably at least 6 %, preferably at least 7 %, preferably at least 8 %, preferably at least 9 %, most preferred at least 10 %, at least 15 %, at least 20% or more haploid progeny. Thereby, a wildtype plant is preferably a plant of the same species which does not comprise the at least one mutation of the plant according to the present invention within the corresponding endogenous CENH3 gene, i.e. the plant is able to express the native CENH3 protein, and a plant expressing wildtype CENH3 is preferably a plant of the same species which comprises i) a nucleotide sequence encoding the CENH3 protein without the at least one mutation of the plant according to the present invention and is able to express said native CENH3 protein or ii) a nucleotide sequence encoding a CENH3 protein from another plant species that shows a comparable functionality to the native CENH3, for instance, such CENH3 protein derived from another plant species can be introduced as a transgene.

Thus, the present invention most advantageously provides means and methods to generate haploid inducer lines in a wide range of eudicot, dicot and monocot species. The present invention also allows the exchange of maternal cytoplasm and to create for instance cytoplasmic male sterility plants with a desired genotype in a single process step. The present invention is advantageous insofar as a single amino acid mutation can be generated by mutagenesis or any other non-GMO-based approaches.

Thus, the entire process of haploidization via application of a haploid inducer line characterized by a point mutated endogenous CENH3 gene encoding a CENH3 protein with amino acid substitutions at at least one of the positions provided by the present invention is non-transgenic in a preferred embodiment.

In the context of the present invention, an "endogenous" gene, allele or protein refers to a non-recombinant sequence of a plant as the sequence occurs in the respective plant, in particular wildtype plant. The term "mutated" refers to a human-altered sequence. Examples of human-induced non-transgenic mutation include exposure of a plant to a high dose of chemical, radiological, or other mutagen for the purposes of selecting mutants. Alternatively, human-induced transgenic mutations, i.e. recombinant alterations or genomic engineering for example by means of TALE nucleases, zinc-finger nucleases or a CRISPR/Cas system, include fusions, insertions, deletions, and/or changes to the DNA or amino acid sequence.

A polynucleotide or polypeptide sequence is "heterologous or exogenous to" an organism if it originates from a foreign species, or, if from the same species, is modified from its original form. "Recombinant" refers to a human-altered, i.e. transgenic polynucleotide or polypeptide sequence. A "transgene" is used as the term is understood in the art and refers to a, preferably heterologous, nucleic acid introduced into a cell by human molecular manipulation of the cell's genome, e.g. by molecular transformation. Thus, a "transgenic plant" is a plant comprising a transgene, i.e. is a genetically-modified plant. The transgenic plant can be the initial plant into which the transgene was introduced as well as progeny thereof whose genome contains the transgene as well.

The term `nucleotide sequence encoding' refers to a nucleic acid which directs the expression of a specific protein, in particular the CENH3 protein or parts thereof. The nucleotide sequences include both the DNA strand sequence that is transcribed into RNA and the RNA sequence that is translated into the protein. The nucleotide sequences include both the full length nucleic acid sequences as well as non-full length sequences derived from the full length sequences.

The term 'gene' refers to a coding nucleotide sequence and associated regulatory nucleotide sequences.

The term `regulatory element' refers to a sequence, preferably a nucleotide sequence, located upstream (5'), within and/or downstream (3') to a nucleotide sequence, preferably a coding sequence, whose transcription and expression is controlled by the regulatory element, potentially in conjunction with the protein biosynthetic apparatus of the cell. `Regulation' or 'regulate' refer to the modulation of the gene expression induced by DNA sequence elements located primarily, but not exclusively upstream (5') from the transcription start of the gene of interest. Regulation may result in an all or none response to a stimulation, or it may result in variations in the level of gene expression.

A regulatory element, in particular DNA sequence, such as a promoter is said to be "operably linked to" or "associated with" a DNA sequence that codes for a RNA or a protein, if the two sequences are situated and orientated such that the regulatory DNA sequence effects expression of the coding DNA sequence.

A 'promoter' is a DNA sequence initiating transcription of an associated DNA sequence, in particular being located upstream (5') from the start of transcription and being involved in recognition and being of the RNA-polymerase. Depending on the specific promoter region it may also include elements that act as regulators of gene expression such as activators, enhancers, and/or repressors.

A '3' regulatory element' (or '3' end') refers to that portion of a gene comprising a DNA segment, excluding the 5' sequence which drives the initiation of transcription and the structural portion of the gene, that determines the correct termination site and contains a polyadenylation signal and any other regulatory signals capable of effecting messenger RNA (mRNA) processing or gene expression. The polyadenylation signal is usually characterised by effecting the addition of polyadenylic acid tracts to the 3' end of the mRNA precursor. Polyadenylation signals are often recognised by the presence of homology to the canonical form 5'-AATAAA-3'.

The term 'coding sequence' refers to that portion of a gene encoding a protein, polypeptide, or a portion thereof, and excluding the regulatory sequences which drive the initiation or termination of transcription.

The gene, coding sequence or the regulatory element may be one normally found in the cell, in which case it is called 'autologous' or 'endogenous', or it may be one not normally found in a cellular location, in which case it is termed 'heterologous', `transgenic' or 'transgene'.

A 'heterologous' gene, coding sequence or regulatory element may also be autologous to the cell but is, however, arranged in an order and/or orientation or in a genomic position or environment not normally found or occurring in the cell in which it is transferred.

The term 'vector' refers to a recombinant DNA construct which may be a plasmid, virus, autonomously replicating sequence, an artificial chromosome, such as the bacterial artificial chromosome BAC, phage or other nucleotide sequence, in which at least two nucleotide sequences, at least one of which is a nucleic acid molecule of the present invention, have been joined or recombined. A vector may be linear or circular. A vector may be composed of a single or double stranded DNA or RNA.

The term 'expression' refers to the transcription and/or translation of an endogenous gene or a transgene in plants.

'Transformation', 'transforming' and 'transferring' refers to methods to transfer nucleic acid molecules, in particular DNA, into cells including, but not limited to, biolistic approaches such as particle bombardment, microinjection, permeabilising the cell membrane with various physical, for instance electroporation, or chemical treatments, for instance polyethylene glycol or PEG, treatments; the fusion of protoplasts or Agrobacterium tumefaciens or rhizogenes mediated trans-formation. For the injection and electroporation of DNA in plant cells there are no specific requirements for the plasmids used. Plasmids such as pUC derivatives can be used. If whole plants are to be regenerated from such transformed cells, the use of a selectable marker is preferred. Depending upon the method for the introduction of desired genes into the plant cell, further DNA sequences may be necessary; if, for example, the Ti or Ri plasmid is used for the transformation of the plant cell, at least the right border, often, however, the right and left border of the Ti and Ri plasmid T-DNA have to be linked as flanking region to the genes to be introduced. Preferably, the transferred nucleic acid molecules are stably integrated in the genome or plastome of the recipient plant.

In the context of the present invention the term `biological activity of a haploid inducer' or 'haploid inducer' or 'haploid inducer line' refers to a plant or plant line having the capability to produce haploid progeny or offspring in at least 0.1 %, at least 0.2 %, 0.3 %, 0.4 %, 0.5 %, 0.6 %, 0.7 %, 0.8 %, 0.9 %, preferably at least 1 %, preferably at least 2 %, preferably at least 3 %, preferably at least 4 %, preferably at least 5 %, preferably at least 6 %, preferably at least 7 %, preferably at least 8 %, preferably at least 9 %, most preferred at least 10 %, most preferred at least 15 %, most preferred at least 20 % of cases when crossed to a wildtype plant or a plant at least expressing wildtype CENH3 protein. Since the chromosomes of the haploid inducer are eliminated during meiosis the resulting haploid progeny only comprises the chromosomes of the wildtype parent. However, in case the haploid inducer was the ovule parent of the cross, the haploid progeny possesses the cytoplasm of the inducer and the chromosomes of the wildtype parent.

The term 'plant' according to the present invention includes whole plants or parts of such a whole plant.

Whole plants preferably are seed plants, or a crop. Parts of a plant are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, trichomes and the like; and progeny of the same.

In any case, the plant of the present invention comprises at least one cell comprising a nucleotide sequence encoding a centromere histone H3 protein comprising a CATD domain, wherein the nucleotide sequence comprises a mutation causing an amino acid substitution at (a) position 92 of SEQ ID NO: 34; (b) position 106 of SEQ ID NO: 44; or (c) position 4 of SEQ ID NO: 1, wherein the amino acid substitution confers the biological activity of a haploid inducer. Most preferably, most or in particular all cells of the plant of the present invention comprises the mutation as described herein.

The species of plants that can be used in the method of the invention are preferably eudicot, dicot and monocot plants.

The term 'plant' in a preferred embodiment relates solely to a whole plant, i.e. a plant exhibiting the full phenotype of a developed plant and capable of reproduction, a developmental earlier stage thereof, e.g. a plant embryo, or to both.

In an embodiment of the present invention the term 'plant' refers to a part of a whole plant, in particular plant material, plant cells or plant cell cultures.

The term `plant cell' describes the structural and physiological unit of the plant, and comprises a protoplast and a cell wall. The plant cell may be in form of an isolated single cell, such as a stomatal guard cells or a cultured cell, or as a part of a higher organized unit such as, for example, a plant tissue, or a plant organ.

The term `plant material' includes plant parts, in particular plant cells, plant tissue, in particular plant propagation material, preferably leaves, stems, roots, emerged radicles, flowers or flower parts, petals, fruits, pollen, pollen tubes, anther filaments, ovules, embryo sacs, egg cells, ovaries, zygotes, embryos, zygotic embryos per se, somatic embryos, hypocotyl sections, apical meristems, vascular bundles, pericycles, seeds, roots, cuttings, cell or tissue cultures, or any other part or product of a plant.

Thus, the present invention also provides plant propagation material of the plants of the present invention. Said "plant propagation material" is understood to be any plant material that may be propagated sexually or asexually in vivo or in vitro. Particularly preferred within the scope of the present invention are protoplasts, cells, calli, tissues, organs, seeds, embryos, pollen, egg cells, zygotes, together with any other propagating material obtained from transgenic plants. Parts of plants, such as for example flowers, stems, fruits, leaves, roots originating in mutated plants or their progeny previously mutated, preferably transformed, by means of the methods of the present invention and therefore consisting at least in part of mutated cells, are also an object of the present invention.

Preferably, the plant according to the present invention is selected from the group consisting of barley *(Hordeum vulgare),* sorghum (*Sorghum bicolor*)*,* rye (*Secale cereale*)*,* Triticale, sugar cane (*Saccharum officinarium*)*,* maize (*Zea mays*), foxtail millet (*Setaria italic*)*,* rice (*Oryza sativa*)*, Oryza minuta, Oryza australiensis, Oryza alta,* wheat (*Triticum aestivum*)*, Triticum durum, Hordeum bulbosum,* purple false brome (*Brachypodium distachyon*)*,* sea barley (*Hordeum marinum*), goat grass (*Aegilops tauschii*)*,* apple (*Malus domestica*)*, Beta vulgaris,* sunflower (*Helianthus annuus*)*,* Australian carrot (*Daucus glochidiatus*)*,* American wild carrot (*Daucus pusillus*)*, Daucus muricatus,* carrot (*Daucus carota*)*,* eucalyptus (Eucalyptus grandis), Erythranthe guttata, Genlisea aurea, woodland tobacco (Nicotiana sylvestris), tobacco (Nicotiana tabacum), Nicotiana tomentosiformis, tomato (Solanum lycopersicum), potato (Solanum tuberosum), coffee (Coffea canephora), grape vine (Vitis vinifera), cucumber (Cucumis sativus), mulberry (Morus notabilis), thale cress (Arabidopsis thaliana), Arabidopsis lyrata, sand rock-cress (Arabidopsis arenosa), Crucihimalaya himalaica, Crucihimalaya wallichii, wavy bittercress (Cardamine flexuosa), peppergrass (Lepidium virginicum), shepherd's-purse (Capsella bursa-pastoris), Olmarabidopsis pumila, hairy rockcress (Arabis hirsuta), rape (Brassica napus), broccoli (Brassica oleracea), Brassica rapa, Brassica juncacea, black mustard (Brassica nigra), radish (Raphanus sativus), Eruca vesicaria sativa, orange (Citrus sinensis), Jatropha curcas, Glycine max, and black cottonwood (Populus trichocarpa).

Particularly preferred the plant is selected from the group consisting of barley *(Hordeum vulgare),* sorghum (*Sorghum bicolor*)*,* rye (*Secale cereale*)*,* Triticale, sugar cane (*Saccharum officinarium*)*,* maize (*Zea mays*)*,* rice (*Oryza sativa*)*,* wheat (*Triticum aestivum*)*, Triticum durum, Avena sativa, Hordeum bulbosum, Beta vulgaris,* sunflower (*Helianthus annuus*)*,* carrot (*Daucus carota*)*,* tobacco (*Nicotiana tabacum*)*,* tomato (*Solanum lycopersicum*)*,* potato (*Solanum tuberosum*)*,* coffee (*Coffea canephora*)*,* grape vine (*Vitis vinifera*)*,* cucumber (*Cucumis sativus*)*,* thale cress (*Arabidopsis haliana*)*,* rape (*Brassica napus*)*,* broccoli (*Brassica oleracea*)*, Brassica rapa, Brassica juncacea,* black mustard (*Brassica nigra*)*,* radish (Raphanus sativus), and *Glycine max.*

The plant according to the present invention contains in a preferred embodiment the nucleotide sequence encoding the CENH3 either as an endogenous gene or a transgene.

The invention relates in a preferred embodiment to a plant according to the present teaching, wherein the at least one amino acid substitution is introduced into the nucleotide sequence encoding CENH3 non-transgenically or transgenically.

Thus, preferably in an embodiment, wherein the at least one mutation is effected in the endogenous CENH3 gene, the obtained plant is non-transgenic. Preferably, the mutation is effected via non-transgenic mutagenesis, in particular chemical mutagenesis, preferably via EMS (ethylmethane sulfonate)-induced TILLING.

Thus, the present invention relates to a plant, wherein the non-transgenic introduction of the at least one mutation causing in the CATD domain an amino acid substitution which confers the biological activity of a haploid inducer is effected via chemical mutagenesis, in particular via TILLING.

In another preferred embodiment, the at least one mutation is introduced into the plant in form of a transgene. Preferably, this is done by transforming a vector comprising a nucleotide sequence encoding at least the CATD domain of CENH3 comprising at least one amino acid substitution as described herein. Methods for transformation of a plant and introducing a transgene into the genome of a plant are well-known in the prior art.

Thus, in one embodiment a plant is provided, wherein the transgenic introduction of the amino acid substitution or amino substitutions within the CENH3 protein is effected via transformation of a vector comprising a nucleotide sequence encoding at least the α2-helix being positioned in the CATD domain and corresponding to nucleotides from position 379 to position 465 of the CENH3 protein as set forth in SEQ ID No. 38 derived from *Arabidopsis thaliana* but comprising at least one amino acid substitution at position 4 of SEQ ID NO: 1, wherein the amino acid substitution confers the biological activity of a haploid inducer. In another embodiment a plant is provided, wherein the introduction of the amino acid substitution or amino substitutions within the CENH3 protein is effected via transformation of a vector comprising a nucleotide sequence encoding at least the CATD domain or a CENH3 protein comprising the CATD domain comprising at least one amino acid substitution at position 4 of SEQ ID NO: 1, wherein the amino acid substitution confers the biological activity of a haploid inducer.

Preferably, the Agrobacterium mediated transformation, floral dip method or particle bombardment are used for transformation.

In the preferred embodiment, wherein the nucleotide sequence encoding the mutated CENH3 protein according to the present invention is transformed into the plant in form of a transgene and one or two alleles of the endogenous CENH3 gene are preferably inactivated or knocked out. Another preferred embodiment, wherein the nucleotide sequence encoding the mutated CENH3 protein according to the present invention is transformed into the plant in form of a transgene and the transgene is overexpressed in order to be more competitive as the endogenous CENH3 protein and preferred during generation of a kinetochore complex.

In a particularly preferred embodiment, the amino acid leucine at position 4 of the α2-helix is substituted in the plant according to the present invention. In particular, the amino acid leucine at position 4 is preferably substituted for isoleucine or phenylalanine.

The present invention also provides a plant obtainable, in particular obtained, by a method according to the present invention and which is characterized by having the biological activity of a haploid inducer.

In a preferred embodiment of the present invention, the method of producing the plant having biological activity of a haploid inducer according to the present invention is not an essentially biological method.

Further, there is provided a method of generating a plant having biological activity of a haploid inducer according to the present invention, comprising the steps of:
i) subjecting seeds of a plant to a sufficient amount of the mutagen ethylmethane sulfonate (EMS) to obtain M1 plants,
ii) allowing sufficient production of fertile M2 plants,
iii) isolating genomic DNA of M2 plants and
iv) selecting individuals possessing at least one amino acid substitution in the CATD domain of CENH3.

The present invention further relates in a preferred embodiment to a method of generating a plant having biological activity of a haploid inducer according to the present invention, comprising the steps of:
a) transforming a plant cell with a nucleotide sequence encoding at least the CATD domain of a CENH3 protein comprising a mutation causing an amino acid substitution at (a) position 92 of SEQ ID NO: 34; (b) position 106 of SEQ ID NO: 44; or (c) position 4 of SEQ ID NO: 1 or a vector comprising a nucleotide sequence encoding at least the CATD domain of a CENH3 protein comprising a mutation causing an amino acid substitution at (a) position 92 of SEQ ID NO: 34; (b) position 106 of SEQ ID NO: 44; or (c) position 4 of SEQ ID NO: 1, and
b) regenerating a plant having the biological activity of a haploid inducer from the plant cell.

Disclosed is that the identified haploid plant can be converted into a double haploid plant, preferably via colchicine treatment. In particular, the present methods do not rely solely on, in particular do not consist of, natural phenomena such as crossing or selection, but in fact are essentially based on the technical teaching so as to provide a specifically mutated nucleotide sequence prepared by mankind's contribution. Thus, the present invention introduces a specific structural feature, namely a mutation, into a nucleotide sequence and a plant of the present disclosure, which mutation is not caused by or associated with any natural phenomena such as crossing or selection.

Further disclosed is that the plant according to the present invention can also be used in a method to restore male fertility by providing a normal cytoplasm to a crossing partner that is CMS. Through such a cross the chromosomes of the CMS plant are introduced into the normal cytoplasm of the haploid inducer of the present invention which is not CMS. However, pollen production of the CMS plant has to be induced via temperature, light, length of day etc.

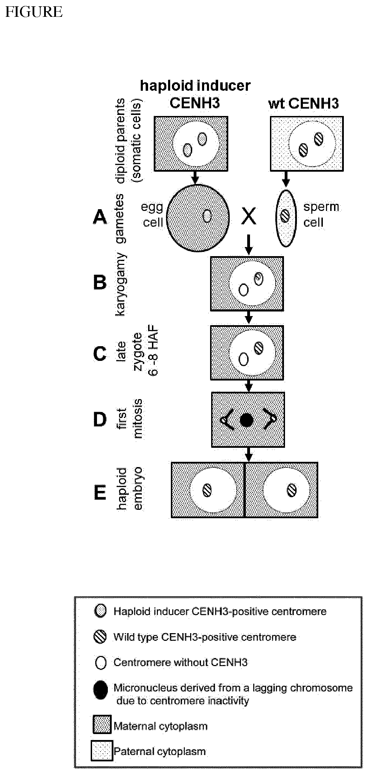

Without being bound by theory a possible model of how the present methods, in particular a method of uniparental chromosome elimination, works in *inducer CENH3* × *wild type CENH3* interspecific hybrid embryos is given in the figure. (A) Likely haploid inducer-derived egg cells contain either less CENH3 or compared to wild type a reduced unknown `CENH3-transgeneration required signature'. A reduced amount of maternal CENH3 is less likely as according to studies performed with a CENH3-GFP reporter in *A. thaliana* plants sperm nuclei but not eggs cells are marked by CENH3. However, it is still possible that residual maternal CENH3s, generating a `centromeric imprinting' are transmitted to the progeny. (B) Within a few hours after fertilization also paternal wild type CENH3 is actively removed from the zygote nucleus, and (C) centromeric reloading of CENH3-GFP in the zygote occurs at the 16-nuclei stage of endosperm development in *A. thaliana.* (D) In embryos undergoing haploidization centromeric reloading of the maternal chromosomes is impaired or delayed causing lagging chromosomes because of centromere inactivity during anaphase. Subsequently micronucleated *haploid inducer* chromosomes will degrade and (E) a haploid embryo will develop. Haploid embryos contain paternal-derived chromosomes in the background of maternal-derived cytoplasm.

The present invention also relates to a nucleotide sequence encoding at least the CATD domain of a CENH3 protein comprising a mutation causing an amino acid substitution at (a) position 92 of SEQ ID NO: 34; (b) position 106 of SEQ ID NO: 44; or (c) position 4 of SEQ ID NO: 1.

The present invention also relates to a vector, in particular viral vector, construct or plasmid comprising said nucleotide sequence and, if present, associates sequences, preferably as indicated herein.

In a particularly preferred embodiment of the present invention, the nucleotide sequence encoding at least the CATD domain of a CENH3 protein preferably comprises at least the complete coding region of CENH3, in particular the gene of CENH3.

In a furthermore preferred embodiment of the present invention, the coding sequence of the CENH3 may be associated with regulatory elements, such as 5'- and/or 3'- regulatory elements, most preferably with a promoter, preferably a constitutive or inducible promoter.

Further, a plant cell comprising said nucleotide sequence or a vector comprising it as a transgene is provided by the present invention.

In the context of the present invention, the term 'comprising' as used herein is understood as to have the meaning of 'including' or 'containing', which means that in addition to the explicitly mentioned element further elements are possibly present.

In a preferred embodiment of the present invention, the term 'comprising' as used herein is also understood to mean `consisting of thereby excluding the presence of other elements besides the explicitly mentioned element.

In a furthermore preferred embodiment, the term 'comprising' as used herein is also understood to mean 'consisting essentially of thereby excluding the presence of other elements providing a significant contribution to the disclosed teaching besides the explicitly mentioned element.

Further preferred embodiments of the present invention are the subject-matter of the subclaims.

The invention will now be described in some more detail by way of the non-limiting examples and a figure.

The sequence protocol shows:
SEQ ID No. 1: the amino acid consensus sequence of the CENH3 α2-helix,
SEQ ID Nos. 2 to 32: nucleotide sequences of primers used in the present teaching,
SEQ ID No. 33: the cDNA nucleotide sequence of the wildtype β-CENH3 protein of Hordeum vulgare,
SEQ ID No. 34: the amino acid sequence of β-CENH3 of Hordeum vulgare,
SEQ ID No. 35: the cDNA sequence of the β-CENH3 of Hordeum vulgare (TILLING line 4528 mutant),
SEQ ID No. 36: the amino acid sequence of β-CENH3 of Hordeum vulgare (TILLING line 4528 mutant),
SEQ ID No. 37: the nucleotide sequence of the wildtype coding sequence (cDNA) of A. thaliana CENH3,
SEQ ID No. 38: the amino acid sequence of the wildtype A. thaliana CENH3,
SEQ ID No. 39: the nucleotide sequence of the coding sequence (cDNA) of the mutated A. thaliana CENH3 (mutant L to I),
SEQ ID No. 40: the amino acid sequence of the mutated A. thaliana CENH3 (mutant L to I),
SEQ ID No. 41: the nucleotide sequence of the coding sequence (cDNA) of the mutated A. thaliana CENH3 (mutant L to F),
SEQ ID No. 42: the amino acid sequence of the mutated A. thaliana CENH3 (mutant L to F),
SEQ ID No. 43: the nucleotide sequence of the wildtype coding sequence (cDNA) of Beta vulgaris CENH3,
SEQ ID No. 44: the amino acid sequence of the wildtype Beta vulgaris CENH3,
SEQ ID No. 45: the nucleotide sequence of the coding sequence (cDNA) of Beta vulgaris CENH3 (mutant L to F),
SEQ ID No. 46: the amino acid sequence of the mutated Beta vulgaris CENH3 (mutant L to F),
SEQ ID No. 47: the nucleotide sequence of the coding sequence (cDNA) of Beta vulgaris CENH3 (mutant L to I) and
SEQ ID No. 48: the amino acid sequence of the mutated Beta vulgaris CENH3 (mutant L to I).

The figure shows schematically a mechanistic model relating to methods of the present invention.

### Examples

### Example 1: Mutagenesis of barley α and βCENH3 by Targeting Local Lesions IN Genomes (TILLING)

To identify whether a single point mutation of endogenous CENH3 could result in a haploid inducer an EMS-induced TILLING population of diploid barley *(Hordeum vulgare)* (Gottwald et al., 2 (2009), BMC Res Notes, 258), a species encoding two functional variants of CENH3 (α and βCENH3) (Sanei et al., 108 (2011), Proc Natl Acad Sci USA, E498-505) was screened. Assuming the complementation of either CENH3 variant a functional mutation of αCENH3 or βCENH3 would still allow the generation of offspring.

To do this, a TILLING population of 10,279 EMS treated diploid barley *(Hordeum vulgare)* plants of cv. *Barke* to identify mutant alleles of α and βCENH3 was screened. Four and three primer combinations
Hv_aCENH3_EX1+2+3_F: AGGCAGGGTCTCAATTCCTT (SEQ ID No. 2),
Hv_aCENH3_EX1+2+3_R: GTCCCATCATCCATCGTCTT (SEQ ID No. 3),
Hv_aCENH3_EX4+5_F: CCCACTTCCTTGTTGTGGAC (SEQ ID No. 4), Hv_aCENH3_EX4+5_R:
   GGCGATAAATGTATCTTGCATTC (SEQ ID No. 5), Hv_aCENH3 EX6 F:
   TGGTAGCAACCAGAGCTACG (SEQ ID No. 6), Hv_aCENH3_EX6_R:
   ACTGGCATGTTTCCTTCTGC (SEQ ID No. 7), Hv_aCENH3 EX7 F:
   CGGACGGAGGGAGTATTTCT (SEQ ID No. 8), Hv_aCENH3_EX7 R:
   GGACATGCCCAAAGAAAGTG (SEQ ID No. 9), Hv_bCENH3_EX1+2_F:
   GCCAGCGAGTACTCCTACAAG (SEQ ID No. 10), Hv_bCENH3_EX1 R:
   TTGAGTTACCAGCCACCACTC (SEQ ID No. 11), Hv_bCENH3_EX3 F:
   GTCATGCACTGTGTCTTGCA (SEQ ID No. 12), Hv bCENH3EX3 R:
   TGCTAAGATCGGATAACTGTGG (SEQ ID No. 13), Hv_bCENH3_EX4 F:
   TGCTCCTGAACAAACTGAACC (SEQ ID No. 14), Hv_bCENH3_EX4 R:
   GTGGCCGTCAGTACAATCG (SEQ ID No. 15)
were used to amplify all exons of the α and β CENH3 variants and parts of the corresponding introns, respectively, by using PCR with a heteroduplex step as described earlier (Gottwald et al., (2009), BMC Res Notes, 258). PCR products were digested with dsDNA Cleavage Kit and analysed using Mutation Discovery Kit and Gel - dsDNA reagent kit on the AdvanCETM FS96 system according to manufacturer's guidelines (Advanced Analytical, IA, USA).

### RNA extraction, PCR and quantitative real time RT-PCR

Total RNA was isolated from roots, leaves using the Trizol method (Chomczynski and Sacchi, 162 (1987), Anal Biochem, 156-159) from anthers (microscopically staged between meiosis and development of mature pollen), carpel, endosperm and embryo by Picopure RNA isolation kit (Arcturus) according to manufacturer. The absence of genomic DNA contamination was confirmed by PCR using GAPDH primers (see Table 2). 10 µl of PCR mixture contained 1 µl of cDNA template, 5 µl of 2× Power SYBR Green PCR Master Mix (Applied Biosystems), 0.33 mM of the forward and reverse primers for each gene (see Table 2). Reactions were run in an Applied Biosystems 7900HT Fast Real-Time PCR System. The PCR was performed using the following conditions: 95°C for 10 min, followed by 40 cycles at 95°C for 15 s, at the annealing temperature of 60°C for 60 s. Three technical replicates were performed for each cDNA sample. Fast Real-Time PCR System and data were analyzed with SDS software v 2.2.2. Transcript levels of each gene were normalized to GAPDH by the following formula: R = 2^^{(-(CtGOI-CtH))*100}, where R = relative changes, GOI = gene of interest, and H = housekeeping (GAPDH). The specificity and efficiency of both primers were determined by qRT-PCR using a dilution series of plasmids of cloned full length barley α and βCENH3 genes. A similar *Ct* value (the PCR cycle at which the fluorescent signal of reporter dye exceeds background level) for equal amount of plasmid indicates that both primers can amplify specific transcripts with the same efficiency.

**Table 2**

| Primer name | Sequence (5' to 3') |
|---|---|
| GAPDH-F | CAATGATAGCTGCACCACCAACTG (SEQ ID No. 21) |
| GAPDH-R | CTAGCTGCCCTTCCACCTCTCCA (SEQ ID No. 22) |
| Hvα-F | AGTCGGTCAATTTTCTCATCCC (SEQ ID No. 23) |
| Hvα-R | CTCTGTAGCCTCTTGAACTGC (SEQ ID No. 24) |
| Hvβ-F | GCCATTGTCGAACAAGAAGG (SEQ ID No. 25) |
| Hvβ-R | TAACACGGTGCGAATGAATG (SEQ ID No. 26) |
| CH3A+L130_F_for | phos-GACAGCTGAAGCATTTGTTGCTCTTC (SEQ ID No. 27) |
| CENH3L130_I_for | phos-GACAGCTGAAGCTATTGTTGCTCTTC (SEQ ID No. 28) |
| CENH3L130_F+I_rev | phos-CAACGATTGATTTGGGGAGGG (SEQ ID No. 29) |
| cenh3-1_mut_for | GGTGCGATTTCTCCAGCAGTAAAAATC (SEQ ID No. 30) |
| cenh3-1_mut_rev | CTGAGAAGATGAAGCACCGGCGATAT (SEQ ID No. 31) |
| cenh3-1_mut2429r | AACTTTTGCCATCCTCGTTTCTGTT (SEQ ID No. 32) |

Only missense point mutations were identified for both barley CENH3 variants.

The non-functionality of mutated CENH3s of homozygous M2 mutants was tested by immunostaining of the centromeres with CENH3 variant-specific antibodies. Mitotic and meiotic chromosomes of *H. vulgare* wildtype and homozygous TILLING line 4528 (plant according to the present invention) have been subjected to immunostaining with antibodies specific for αCENH3 and βCENH3. αCENH3 and βCENH3 signals at centromeres were revealed in all mutants, while only the homozygous TILLING line 4528 which contains a leucine to phenylalanine substitution at amino acid 92 (SEQ ID No. 36), i.e. amino acid position 4 of the consensus sequence SEQ ID No. 1, displayed no centromeric βCENH3 signals in mitotic, meiotic and interphase cells. The leucine to phenylalanine substitution at amino acid 92 of SEQ ID No. 36 of the *H. vulgare* β-CENH3 sequence corresponds to a single nucleotide substitution from C to T at position 274 of the *H. vulgare* β-CENH3 cDNA sequence (SEQ ID NO: 35).

Only weak dispersed βCENH3 signals outside centromeres were found in this line. Transcription levels of α and βCENH3 in wildtype (cv Barke) (SEQ ID no. 33 and 34) and TILLING line 4528 with mutated βCENH3 have been measured. The relative expression level of α and βCENH3 was measured in different tissues using specific primers (Table 2). cDNA was prepared from total RNA and gene expression levels were normalized to the expression level of glyceryl phosphate dehydrogenase (GRPTA). Obviously, the centromeric loading of the mutated βCENH3 variant seems to be impaired, while no different transcription level between wild type and mutated βCENH3 was found. Hence, centromeres exclusively composed of αCENH3 are sufficient for mitotic centromere function in barley as no obvious chromosome segregation defects, such as anaphase bridges, as well as changes of ploidy or cycle vales was found. In addition, no obvious changes of the plant habitus were observed in mutant plants. In particular, no significant differences in phenotype, ploidy levels, cycle values and growth phenotype between homozygous plants of TILLING line 4528 and barley wildtype could be detected.

The issue was addressed whether missing βCENH3 is compensated by additional αCENH3 to maintain the centromere function in the mutant. Therefore, αCENH3 immunostaining signals of wildtype (126 centromeres measured) and of line 4528 (56 centromeres measured) were comparatively quantified by pixel intensity measurements. An increase of 19.8% αCENH3 in the mutant indicates that the missing βCENH3 is partly compensated by additionally incorporated αCENH3. The βCENH3 mutation is located in an evolutionarily highly conserved targeting domain (CATD) defined by parts of α1helix, loop 1 and α2helix of the histone fold. This domain is required for centromere loading of CENH3 by chaperons.

### Indirect immunostaining

Indirect immunostaining of nuclei and chromosomes was carried out as described previously (Sanei et al., 108 (2011), Proc Natl Acad Sci USA, E498-505). CENH3 of barley was detected with guenia pig anti-αCENH3-specific and rabbit anti-βCENH3-specific antibodies. A rabbit HTR12-specific antibody (abcam, ab72001) was used for the detection of *A. thaliana* CENH3 (AtCENH3). Epifluorescence signals were recorded with a cooled CCD-camera (ORCA-ER, Hamamatsu). Imaging was performed by using an Olympus BX61 microscope and an ORCA-ER CCD camera (Hamamatsu). To analyse the structures of immunosignals and chromatin at an optical resolution of -100 nm (super-resolution) Structured Illumination Microscopy (SIM) was applied using a C-Apo 63x/1.2W Korr objective of an Elyra microscope system and the software ZEN (Zeiss, Germany). Images were captured separately for each fluorochrome using appropriate excitation and emission filters. The images were merged using the Adobe Photoshop 6.0 software. To determine the amount of α and βCENH3 in nuclei fluorescence intensities were measured using the TINA 2.0 software in maximum intensity projections generated from stacks of optical SIM sections through the specimens by the ZEN software. An intensity threshold was set to computationally subtract the background pixels from the image. The corrected sum of grey values of all signals within the nucleus was used to determine the CENH3 content. 3D-rendering based on SIM image stacks was done using the ZEN software.

### Example 2: Arabidopsis thaliana

To proof whether the mutation in the CATD domain caused the observed impaired centromere loading, eYFP was N-terminally fused to the coding sequence (CDS) of *A. thaliana* CENH3 (SEQ ID No. 37, protein: SEQ ID No. 38) with an L/I (leucine/isoleucine) (CDS: SEQ ID No. 39, protein SEQ ID No. 40) or L/F (leucine/phenylalanine) (CDS: SEQ ID No. 41, protein SEQ ID No. 42) exchange of the corresponding positions (L130I or L130F, corresponding to amino acid position 92 in βCENH3 of barley, i.e. amino acid position 4 of the consensus sequence SEQ ID No. 1) in A. thaliana CENH3. The leucine to isoleucin substitution at position 130 of *A. thaliana* corresponds to a single nucleotide substitution from C to A at position 388 of SEQ ID no. 37.

The amino acid substitution from leucine to phenylalanine at position 130 is caused by two nucleotide substitutions, namely TC to AT at positions 387 and 388 of SEQ ID No. 37.

Double labelling of transgenic *A. thaliana* with corresponding anti-wild type CENH3 and anti-GFP revealed a significantly reduced centromere targeting of the mutated CENH3s.

Next, to test for haploid inducer ability *A. thaliana* genomic CENH3 constructs with a L130I or L130F exchange were used to transform heterozygous CENH3 knock-out *A. thaliana* plants (Ravi and Chan, Nature, 464 (2010), 615-618). Genotyping identified homozygous CENH3 null mutants which were complemented with either genomic CENH3 wild type, L130I or L130F constructs. As viable diploid plants containing either of the constructs were obtained, it is likely that this mutation does not impair the centromere function in homozygous *A. thaliana* plants. When CENH3 null mutants expressing a point mutated L130F CENH3 protein were crossed to wild type, chromosomes from the mutant are eliminated, producing haploid progeny. Flow cytometric analysis revealed that 10.7% of the F1 plants were haploid.

### Cloning and generation of CENH3 transgenes

To generate *CENH3* genomic fragments carrying mutations, resulting in phenylalanine 130 (F130) and isoleucine 130 (I130) instead of wild-type leucine 130 (L130), a genomic *CENH3* fragment in pCAMBIA1300 vector used to complement *cenh3-1*/*cenh3-1 (cenh3* null mutant) (Ravi and Chan, Nature, 464 (2010), 615-618; Ravi et al., Genetics, 186 (2010), 461-471) was subcloned via the unique *Hind*III and *Bam*HI sites into pBlueScript II KS (Strategene, www.stratagene.com). Mutations of *CENH3,* L130I or L130F, were generated in pBlueScript II KS using a Phusion^{®} Site-Directed Mutagenesis Kit (Finnzymes, www.finnzymes.com) according to manufacturer's instructions with minor changes as described. Following 5'-phosphorylated primers were used for mutagenesis: CH3A+L130_F_for, CENH3L130_I_for and CENH3L130_F+I_rev. Mutated *CENH3* genomic fragments were subcloned via the unique *Hind*III and *Bam*HI sites into the initial pCAMBIA1300 containing a hygromycin resistance marker. All constructs were verified by sequencing. For primers see Table 2, above.

To generate *p35S::eYFP-CENH3* fusion constructs containing mutations within the *CENH3* CDS, resulting in L130I or L130F, a plasmid (p35S-BM; Schmidt, www.dna-cloning.com) containing a *p35S:.eYFP-CENH3* expression (Lermontova, 18 (2006), Plant Cell, 615-618) was used as template for the Phusion^{®} Site-Directed Mutagenesis Kit (Finnzymes, www.finnzymes.com). Primers and strategies to introduce desired mutations were the same as above. Resulting expression cassettes (35Spro, eYFP-(mutated)*CENH3* and *NOS* terminator) were subcloned via unique *Sfi*I restriction sites into the pLH7000 vector containing a phosphinotricine resistance marker (Schmidt, www.dna-cloning.com) and verified by sequencing.

### Plant transformation, culture conditions and cross-pollination

*A. thaliana* wild-type (SEQ ID No. 37 and 38) and *cenh3-1*/*CENH3* heterozygotes plants (both accession Columbia-0) were transformed by the floral dip method (Clough and Bent, 16 (1998), Plant J, 735-743). Transgenic progenies were selected on Murashige and Skoog solid medium containing the corresponding antibiotic. Plants were germinated on Petri dishes under long-day conditions (20°C 16h light/18°C 8h dark), grown for 4 weeks under short-day conditions (20°C 8h light/18°C 16h dark) and then shifted to long-day conditions again. For crossing, the closed buds of mutant *cenh3 A. thaliana* were emasculated by removing the immature anthers with the help of forceps. The stigmas of emasculated buds were fertilized with the yellowish pollen from mature anthers of freshly opened wild type *A. thaliana* flowers.

### DNA extraction and genotyping of A. thaliana

Genomic DNA preparations and PCR-based genotyping were performed using standard methods. DNA was extracted according to Edwards et al. (1991), Nucleic Acids Res 19, 1349.

Plants were genotyped for *cenh3-1* in a dCAPS genotyping reaction. The dCAPS primers, cenh3-1_mut_for and cenh3-1_mut_rev, were used to amplify *CENH3.* Amplicons were digested with EcoRV and resolved on a gel. *cenh3-1* mutant allele is not cut (215 bp) while the WT *CENH3* allele is cut (191 and 24 bp). For primers see Table 2. To genotype the endogenous *CENH3* locus for *cenh3-1* in the offspring of *cenh3-1*/*CENH3* plants transformed with the *CENH3* genomic locus (untagged *CENH3* transgene with L130, L130I or L130F), an initial PCR reaction was performed with one primer outside of the transgene *CENH3* locus, allowing specific amplification of the endogenous and not the transgenic *CENH3* locus. Primers used were cenh3-1_mut_for and cenh3-1_mut2320r/cenh3-1_mut2429r. Amplicons were purified and used as template for a second dCAPS PCR genotyping reaction as described above for *cenh3-1* plants. For Primers see Table 2. Presence of transgene was verified by PCR.

### Flow cytometric analysis of plants and seeds

For flow cytometric ploidy analyses of plants equal amounts of leaf material of 5 to 10 individuals were chopped simultaneously in nuclei isolation buffer (Galbraith et al. (1983), Science 220, 1049-1051) supplemented with DNas-free RNase (50 µg/ml) and propidium iodide (50 µg/ml) with a sharp razor blade. The nuclei suspensions were filtered through 35 µm cell strainer cap into 5 ml polystyrene tubes (BD Biosciences) and measured on a FACStar^{PLUS} cell sorter (BD Biosciences) equipped with an argon ion laser INNOVA 90C (Coherent). Approximately 10,000 nuclei were measured and analysed using the software CELL Quest ver. 3.3 (BD Biosciences). The resulting histograms were compared to a reference histogram representing a diploid wild type plant. In cases where an additional peak at the haploid position was detected, the plants were individually measured again to identify the haploid individuals.

Nuclei isolation of seeds was performed as described above using the nuclei isolation buffer MA VI (100 mM Tris-HCl, 5.3 mM MgCl₂, 86 mM NaCl, 30.6 mM sodium citrate, 1.45 mM Triton X-100, pH 7.0; supplemented with 50 µg/ml DNas-free RNase and 50 µg/ml propidium iodide). Nuclei suspensions were measured on a FACSAria cell sorter (BD Biosciences) and analysed using the FACS Diva software ver. 6.1.3 (BD Biosciences). Similarly as above, first 10 to 20 seeds were pooled to identify lines with haploid embryos and in a second step single seeds were co-chopped together with leaf material from *Raphanus sativus* (Genebank Gatersleben, accession number: RA 34) as internal reference to confirm the occurrence of haploid seeds.

### Example 3: Beta vulgaris

Further, the functional significance of the identified mutation was assayed also in the sugar beet *Beta vulgaris.* RFP reporter constructs containing the cDNA of Beta vulgaris CENH3 (SEQ ID No. 43, protein SEQ ID No. 44) with an L106F (SEQ ID No. 45, protein SEQ ID No. 46) or L106I (SEQ ID No. 47, protein SEQ ID No. 48) exchange (corresponding to amino acid position 92 of barley, amino acid position 4 of the consensus sequence SEQ ID No. 1) were generated and used for stable transformation of sugar beet and a reduced centromere targeting of the mutated CENH3s was detected.

The amino acid substitution from leucine to phenylalanine at position 106 is caused by two nucleotide substitutions, namely C to T at position 316 and G to T at position 318 of SEQ ID no. 43.

The amino acid substitution from leucine to isoleucine at position 106 is caused by two nucleotide substitutions, namely C to A at position 316 and G to T at position 318 of SEQ ID no. 43.

Plant transformation and culture conditions

*Beta vulgaris* wild-type leaves of 6 - 8 week old plants (grown under semi-controlled greenhouse conditions) were transiently transformed by particle bombardment (300 µg gold coated with 0.5 µg plasmid DNA). Bombarded leaves were incubated for 48 - 72 h (25°C 16h light (350 µmolm⁻²s⁻¹)/ 8h dark) before microscopic analysis. Stable transformation of *B. vulgaris* callus was performed as described in Lindsey & Gallois, 1990 (Journal of experimental botany, 41(5), 529-536) (selection via kanamycin). After approx. 2 month (24°C 16 h light (55 µmolm⁻²s⁻¹) /8 h dark) callus cells were microscopically analysed.

### Cloning and generation of CENH3 transgenes

To generate the *35S::RFP-CENH3* fusion construct, CENH3 was amplified from sugar beet cDNA with the following primers:
BvCENH3-cds1: GGATCCATGAGAGTTAAACACACTGC (SEQ ID No. 16), BvCENH3-cds2: GGATCCTGTTCAGTTACCATCCCCTC (SEQ ID No. 17),
cloned into a vector containing a *35Spro, RFP* and *35S-terminator* expression cassette For constructs containing mutations within the *CENH3* coding sequence, resulting in F106 and I1106 instead of L106, the above mentioned plasmid containing the *35S::RFP-CENH3* expression cassette was used as template for primer based mutagenesis. The *Pst*I site close to the position of the desired mutation was used to split CENH3 into two parts. Via mutations in the Primers the desired mutations were integrated:
BvCENH3_mut_Fw: ATGGATCCATGAGAGTTAAACACACTGC (SEQ ID No. 18),
BvCENH3_L->F_Rv: CTCTGCAGCCTCTTGAAGGGCCATAAAAGC (SEQ ID No. 19),
BvCENH3_L->I_Rv: CTCTGCAGCCTCTTGAAGGGCCATAATAGC (SEQ ID No. 20).

Resulting expression cassettes (35Spro, RFP-(mutated)*CENH3* and *35S-terminator*) were verified by sequencing.

### Analysis of CENH3 binding in B. vulgaris

To analyse the binding of CENH3 and the mutated CENH3 either leaf or callus material was analysed using a C-Apo 63x/1.2W Korr objective of an Axio Imager M2 microscope system and the software ZEN (Zeiss, Germany).

## Claims

1. Plant having biological activity of a haploid inducer and comprising a nucleotide sequence encoding a centromer histone H3 (CENH3) protein comprising a CATD domain, wherein the nucleotide sequence comprises a mutation causing an amino acid substitution at (a) position 92 of SEQ ID NO: 34; (b) position 106 of SEQ ID NO: 44; or (c) position 4 of SEQ ID NO: 1, wherein the amino acid substitution confers the biological activity of a haploid inducer.

2. Plant according to claim 1, wherein the mutation causes a substitution of a specified amino acid of SEQ ID NO: 1 wherein the specified amino acid is L at (a) position 4 of SEQ ID NO: 1; (b) position 92 of SEQ ID NO: 34; or (c) position 106 of SEQ ID NO: 44.

3. Plant according to any of the preceding claims, wherein crossing between the plant and a wildtype plant or plant expressing wildtype CENH3 protein yields at least 0.1 % haploid progeny.

4. Plant according to any of the preceding claims, wherein the nucleotide sequence comprising the mutation is an endogenous gene or a transgene.

5. Plant according to claim 4, wherein the nucleotide sequence comprising the mutation is a transgene and at least one endogenous gene encoding a CENH3 protein is inactivated or knocked out.

6. Plant according to any of the preceding claims, wherein the amino acid leucine at (a) position 4 of SEQ ID NO: 1, (b) position 92 of SEQ ID NO: 34; or (c) position 106 of SEQ ID NO: 44 is substituted, preferably substituted for isoleucine or phenylalanine.

7. Part of the plant according to any of the preceding claims, which is preferably a shoot vegetative organ, root, flower or floral organ, seed, fruit, ovule, embryo, plant tissue or cell, wherein the part of the plant comprises a nucleotide sequence as defined in claim 1.

8. Nucleotide sequence encoding at least the CATD domain of a CENH3 protein comprising a mutation causing an amino acid substitution at (a) position 92 of SEQ ID NO: 34; (b) position 106 of SEQ ID NO: 44; or (c) position 4 of SEQ ID NO: 1.

9. Vector comprising the nucleotide sequence of claim 8.

10. Plant cell or host cell comprising the nucleotide sequence of claim 8 or the vector of claim 9 as a transgene.

11. A method of generating a plant according to claims 1 to 6, comprising the steps of:
a) transforming a plant cell with the nucleotide sequence of claim 8 or the vector of claim 9, and
b) regenerating a plant having the biological activity of a haploid inducer from the plant cell.

## Patentansprüche

1. Pflanze, die die biologische Aktivität eines Haploidinduktors aufweist und eine Nukleotidsequenz umfasst, die für ein Centromer-Histon-H3 (CENH3)-Protein kodiert, das eine CATD-Domäne umfasst, wobei die Nukleotidsequenz eine Mutation umfasst, die einen Austausch der Aminosäure an (a) Position 92 von SEQ ID NO: 34; (b) Position 106 von SEQ ID NO: 44; oder (c) Position 4 von SEQ ID NO: 1 bewirkt, wobei der Austausch einer Aminosäure die biologische Aktivität eines Haploidinduktors verleiht.

2. Pflanze nach Anspruch 1, wobei die Mutation einen Austausch einer spezifizierten Aminosäure von SEQ ID NO: 1 bewirkt, wobei die spezifizierte Aminosäure L ist an (a) Position 4 von SEQ ID NO: 1; (b) Position 92 von SEQ ID NO: 34; oder (c) Position 106 von SEQ ID NO: 44.

3. Pflanze nach einem der vorhergehenden Ansprüche, wobei die Kreuzung zwischen der Pflanze und einer Wildtyp-Pflanze oder einer Pflanze, die Wildtyp-CENH3-Protein exprimiert, mindestens 0,1 % haploide Nachkommenschaft ergibt.

4. Pflanze nach einem der vorhergehenden Ansprüche, wobei die Nukleotidsequenz, die die Mutation umfasst, ein endogenes Gen oder ein Transgen ist.

5. Pflanze nach Anspruch 4, wobei die Nukleotidsequenz, welche die Mutation umfasst, ein Transgen ist, und wobei mindestens ein endogenes Gen, das für ein CENH3-Protein kodiert, inaktiviert oder ausgeknockt ist.

6. Pflanze nach einem der vorhergehenden Ansprüche, wobei die Aminosäure Leucin an (a) Position 4 von SEQ ID NO: 1, (b) Position 92 von SEQ ID NO: 34; oder (c) Position 106 von SEQ ID NO: 44 ausgetauscht ist, vorzugsweise ausgetauscht durch Isoleucin oder Phenylalanin.

7. Teil der Pflanze nach einem der vorhergehenden Ansprüche, der vorzugsweise ein vegetatives Sprossorgan, Wurzel, Blüte oder Blütenorgan, Samen, Frucht, Eizelle, Embryo, Pflanzengewebe oder -zelle ist, wobei der Teil der Pflanze eine Nukleotidsequenz wie in Anspruch 1 definiert umfasst.

8. Nukleotidsequenz, die mindestens die CATD-Domäne eines CENH3-Proteins kodiert, umfassend eine Mutation, die einen Austausch einer Aminosäure bewirkt an (a) Position 92 von SEQ ID NO: 34; (b) Position 106 von SEQ ID NO: 44; oder (c) Position 4 von SEQ ID NO: 1.

9. Vektor, der die Nukleotidsequenz nach Anspruch 8 enthält.

10. Pflanzenzelle oder Wirtszelle, die die Nukleotidsequenz nach Anspruch 8 oder den Vektor nach Anspruch 9 als Transgen enthält.

11. Verfahren zur Erzeugung einer Pflanze nach einem der Ansprüche 1 bis 6, umfassend die folgenden Schritte:
a) Transformieren einer Pflanzenzelle mit der Nukleotidsequenz von Anspruch 8 oder dem Vektor von Anspruch 9, und
b) Regenerieren einer Pflanze mit der biologischen Aktivität eines Haploidinduktors aus der Pflanzenzelle.

## Revendications

1. Plante ayant l'activité biologique d'un inducteur haploïde et comprenant une séquence nucléotidique codant pour une protéine centromère histone H3 (CENH3) comprenant un domaine CATD, dans laquelle la séquence nucléotidique comprend une mutation provoquant une substitution d'acide aminé en (a) position 92 de SEQ ID NO: 34; (b) position 106 de SEQ ID NO: 44; ou (c) position 4 de SEQ ID NO: 1, dans laquelle la substitution d'acide aminé confère l'activité biologique d'un inducteur haploïde.

2. Plante selon la revendication 1, dans laquelle la mutation provoque une substitution d'un acide aminé spécifié de SEQ ID NO: 1, dans laquelle l'acide aminé spécifié est L en (a) position 4 de SEQ ID NO: 1 ; (b) position 92 de SEQ ID NO: 34; ou (c) position 106 de SEQ ID NO: 44.

3. Plante selon l'une quelconque des revendications précédentes, dans laquelle le croisement entre la plante et une plante de type sauvage ou une plante exprimant la protéine CENH3 de type sauvage produit au moins 0,1 % de descendance haploïde.

4. Plante selon l'une quelconque des revendications précédentes, dans laquelle la séquence nucléotidique comportant la mutation est un gène endogène ou un transgène.

5. Plante selon la revendication 4, dans laquelle la séquence nucléotidique comportant la mutation est un transgène et dans laquelle au moins un gène endogène codant pour une protéine CENH3 est inactivé ou knock-out.

6. Plante selon l'une quelconque des revendications précédentes, dans laquelle l'acide aminé leucine en (a) position 4 de SEQ ID NO: 1, (b) position 92 de SEQ ID NO: 34; ou (c) position 106 de SEQ ID NO: 44 est substituée, de préférence substituée par l'isoleucine ou la phénylalanine.

7. Partie de la plante selon l'une quelconque des revendications précédentes, qui est de préférence un organe végétatif de pousse, une racine, une fleur ou un organe floral, une graine, un fruit, un ovule, un embryon, un tissu ou une cellule végétale, dans laquelle la partie de la plante comprend un séquence nucléotidique telle que définie dans la revendication 1.

8. Séquence nucléotidique codant pour au moins le domaine CATD d'une protéine CENH3 comportant une mutation provoquant une substitution d'acide aminé en (a) position 92 de SEQ ID NO: 34; (b) position 106 de SEQ ID NO: 44; ou (c) position 4 de SEQ ID NO: 1.

9. Vecteur comprenant la séquence nucléotidique selon la revendication 8.

10. Cellule végétale ou cellule hôte comprenant la séquence nucléotidique selon la revendication 8 ou le vecteur selon la revendication 9 en tant que transgène.

11. Procédé de génération d'une plante selon les revendications 1 à 6, comprenant les étapes consistant à:
a) transformer une cellule végétale avec la séquence nucléotidique selon la revendication 8 ou le vecteur selon la revendication 9, et
b) régénérer une plante ayant l'activité biologique d'un inducteur haploïde de la cellule végétale.
